# EUROPÄISCHE PATENTANMELDUNG

(11) **EP 0 735 020 A2**
(43) Veröffentlichungstag der Anmeldung: **02.10.1996**
(21) Anmeldenummer: 96104371.8
(22) Anmeldetag: 20.03.1996
(51) Int. Cl.: C07C 303/32, C07C 309/12

(54) **Verfahren zur Herstellung von Acyloxyalkansulfonaten**

(30) Priorität: 29.03.1995 DE 19511459
(71) Anmelder: HOECHST AKTIENGESELLSCHAFT, 65929 Frankfurt am Main (DE)
(72) Erfinder: Bühring, Dirk, Dr., Jardim Natal, 08613-010 Suzano - SP (BR)

(57) **Zusammenfassung**

Die Acyloxyalkansulfonate werden erfindungsgemäß durch Direktveresterung erhalten, wobei man mindestens eine Fettsäure mit mindestens einem Ammonium-Hydroxyalkansulfonat der Formel HO-A-SO₃⁻ ⁺NR¹R²R³R⁴, worin A ein C₂ bis C₄-Alkylen ist und R¹, R², R³ und R⁴ Wasserstoff oder eine C₁ bis C₄-Alkylengruppe sind, in Gegenwart eines Veresterungskatalysators bei einer Temperatur von höchstens 200 °C unter Entfernen des anwesenden Wassers verestert. Man erhält nach diesem Verfahren ein Produkt mit einem hohen Gehalt an Acyloxyalkanammoniumsulfonat, ohne daß es des Zusatzes eines Konsistenzreglers bedarf.

## Beschreibung

Die Erfindung betrifft ein Verfahren zur Herstellung von Acyloxyalkansulfonaten mit verbesserten Eigenschaften durch Veresterung von Fettsäuren mit Hydroxyalkansulfonaten.

Acyloxyalkansulfonate sind wertvolle anionische Tenside, die vor allem zur Bereitung von Syndet-Seifen, kosmetischen Mitteln und Reinigungsformulierungen eingesetzt werden. Ihre Herstellung erfolgt vorteilhaft durch Veresterung von mindestens einer Fettsäure mit mindestens einem Hydroxyalkansulfonat (Direktveresterung). Ein solches Verfahren ist zum Beispiel in EP-A-0 585 071 (US-A-5 384 421) beschrieben. Dabei werden die Fettsäure und das Salz der Hydroxyalkansulfonsäure in Gegenwart eines Veresterungskatalysators und eines Konsistenzreglers bei einer Temperatur von 180 bis 240 °C umgesetzt unter gleichzeitiger Entfernung von vorhandenem Wasser. Als Konsistenzregler werden bestimmte Paraffine eingesetzt. Der Einsatz derartiger Verbindungen ist erforderlich, weil mit fortschreitender Veresterung das Reaktionsgemisch hochviskos wird. Mit Konsistenzreglern wird zwar eine niedrigere Viskosität des Reaktionsgemisches erreicht und die Umsetzung erleichtert, das Veresterungsprodukt enthält aber die eingesetzten Verbindungen, wodurch das angestrebte Acyloxyalkansulfonat mehr oder weniger verdünnt erhalten wird.

Es wurde nun überraschenderweise gefunden, daß man die in Rede stehende Direktveresterung ohne Konsistenzregler durchführen kann und damit ein Veresterungsprodukt mit einem hohen Gehalt an Acyloxyalkansulfonat erhält, wenn man die Fettsäuren mit Hydroxyalkanammoniumsulfonat verestert und eine Veresterungstemperatur von maximal 200 °C einhält. Es ist ein unerwartetes Ergebnis, daß unter den zahlreichen Hydroxyalkansulfonaten gerade die Ammoniumsalze von Hydroxyalkansulfonsäuren mit Fettsäuren ohne Konsistenzregler umgesetzt werden können. Auf diese Weise ist es möglich, hochkonzentrierte Acyloxyalkansulfonate herzustellen, die als weitere unerwartete Eigenschaft eine gute Wasserlöslichkeit aufweisen.

Das erfindungsgemäße verfahren zur Herstellung von Acyloxyalkansulfonaten mit verbesserten Eigenschaften durch Veresterung von Fettsäuren mit Hydroxyalkansulfonaten ist dadurch gekennzeichnet, daß man mindestens eine Fettsäure der Formel 1 RCOOH (1), worin R ein Kohlenwasserstoffrest mit 5 bis 31 Kohlenstoffatomen ist, mit mindestens einem Ammonium-Hydroxyalkansulfonat der Formel 2 HO-A-SO₃⁻ ⁺NR¹R²R³R⁴ (2), worin A ein C₂ bis C₄-Alkylen ist und R¹, R², R³ und R⁴, gleich oder verschieden, Wasserstoff oder eine C₁ bis C₄-Alkylgruppe sind, in Gegenwart eines Veresterungskatalysators und in Abwesenheit eines Konsistenzreglers bei einer Temperatur von höchstens 200 °C unter Entfernen des anwesenden Wassers verestert, wobei ein Produkt mit einem hohen Gehalt an Acyloxyalkanammoniumsulfonat erhalten wird.

Beim erfindungsgemäßen Verfahren wird also ein ausgewähltes Salz der Hydroxyalkansulfonsäure eingesetzt, und zwar ein Ammoniumsalz. Im Ammoniumion, das der nachstehenden Formel 3 entspricht 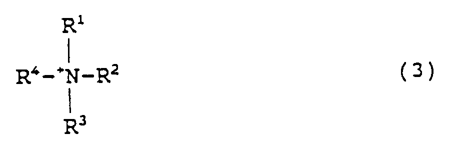 sind R¹ bis R⁴ Wasserstoff oder ein C₁ bis C₄-Alkyl, vorzugsweise Methyl oder Ethyl, wobei R¹ bis R⁴ gleich oder verschieden sein können. So ist das Ammoniumion ⁺NH₄, wenn die Reste R¹ bis R⁴ jeweils Wasserstoff sind, oder ⁺NH(CH₃)₃ oder ⁺NH(C₂H₅)₃, wenn R¹ Wasserstoff ist und R² bis R⁴ Methyl oder Ethyl sind. Der zweiwertige Rest A im erfindungsgemäß einzusetzenden Ammoniumsalz der angegebenen Formel 2 ist vorzugsweise -CH₂CH₂-, -(CH₂)₃- oder -CH₂CH(CH₃)-, wobei Ethylen besonders bevorzugt ist. Das erfindungsgemäß bevorzugte Salz ist demnach Ammonium-Hydroxyethansulfonat (Ammoniumisethionat). Die Ammoniumsalze können als solche eingesetzt werden, bevorzugt werden sie in Form einer wäßrigen Lösung, im allgemeinen als 40 bis 65gew.%ige Lösung, eingesetzt.

Die Fettsäure entspricht der oben angegebenen Formel 1 RCOOH (1), worin R ein Kohlenwasserstoffrest mit 5 bis 31 Kohlenstoffatomen ist, der gesättigt oder ungesättigt, gerade oder verzweigt sein kann, wobei gerade (unverzweigt) bevorzugt ist. R kann auch ein Gemisch von solchen Kohlenwasserstoffresten sein. R ist bevorzugt C₅ bis C₂₁-Alkyl oder C₅ bis C₂₁-Alkenyl oder eine Mischung davon. Die Alkyl- und Alkenylreste sind vorzugsweise unverzweigt. Die Alkenylreste sind ferner vorzugsweise ein- bis dreifach ungesättigt. Als Beispiele von Fettsäuren seien genannt Capronsäure, Caprinsäure, Laurinsäure, Myristinsäure, Stearinsäure, Arachinsäure, Ölsäure, Linolsäure, Linolensäure, Cocosfettsäure und Talgfettsäure sowie Mischungen davon.

Die erfindungsgemäße Umsetzung von Fettsäure und Ammonium-Hydroxyalkansulfonat wird in Gegenwart eines Katalysators durchgeführt. Geeignete Veresterungskatalysatoren sind in der genannten EP-A-0 585 071 ausführlich beschrieben, die hier miteinbezogen wird. Es handelt sich um Alkansulfonsäuren, Hydroxyalkansulfonsäuren, Arylsulfonsäuren, anorganischen Säuren wie Schwefelsäure, Phosphorsäure, phosphorige Säure, Borsäure oder deren Anhydride, Schwermetallsalze wie Zinksulfat, Zirkoniumsulfat, Zinkisethionat, Zinkborat, Aluminiumsulfat, Titansulfat oder Wolframphosphat, Metalloxide wie Zinkoxid, Aluminiumoxid, Magnesiumoxid, Ceroxid, Zirkoniumoxid oder Lanthanoxid, ferner um Mischungen von zwei oder mehreren der genannten Katalysatoren und um Seifen, die aus Schwermetallen und Metalloxiden gebildet werden. Ein besonders bevorzugter Veresterungskatalysator ist Zinkoxid. Der Veresterungskatalysator wird in einer Menge von im allgemeinen 0,05 bis 2 Gew.-%, vorzugsweise 0,05 bis 1 Gew.-%, eingesetzt, Gewichtsprozente bezogen auf Fettsäure und Hydroxyalkansulfonsäure-Ammoniumsalz.

Die Umsetzung von Fettsäure und Hydroxyalkansulfonsäure-Ammoniumsalz, im allgemeinen im Molverhältnis von 1 : 1 bis 2 : 1, vorzugsweise etwa 1 : 1, wird erfindungsgemäß bei einer Temperatur von höchstens 200 °C durchgeführt, das heißt, daß während der gesamten Umsetzung die Temperatur nicht über 200 °C steigen soll. Der bevorzugte Temperaturbereich liegt bei 170 bis 200 °C und der besonders bevorzugte bei 180 bis 190 °C. Das gegebenenfalls mit den Ausgangskomponenten in die Reaktionsmischung eingebrachte Wasser und das durch die Veresterungsreaktion entstehende Wasser wird kontinuierlich aus der Reaktionsmischung ausgetragen. Die Reaktionsmischung ist aufgrund des eingesetzten speziellen Hydroxyalkansulfonsäuresalzes bis zum Ende der Umsetzung, auch bei 100%igem Umsatz, homogen, relativ niedrigviskos und gut rührbar; ein Konsistenzregler ist nicht erforderlich. Die Zeit bis zum angestrebten Umsatz von Fettsäure oder von Ammonium-Hydroxyalkansulfonat liegt bei etwa 4 bis 8 Stunden. Im allgemeinen wird man, zum Beispiel aus Zeitgründen, 100%igen Umsatz nicht anstreben, sondern bei einem niedrigeren Prozentsatz, zum Beispiel bei 75 bis 90 Gew.-% Acyloxyalkanammoniumsulfonat, die Veresterungsreaktion abbrechen.

Das erfindungsgemäße Verfahren kann im einzelnen zum Beispiel in der Weise durchgeführt werden, daß man - bei Atmosphärendruck - die Fettsäure, das Ammoniumsalz der Hydroxyalkansulfonsäure und den Veresterungskatalysator in einem Reaktionsgefäß vorlegt und die Mischung unter Rühren auf die angegebene Temperatur erhitzt. Vorhandenes Wasser destilliert bereits während des Erhitzens der Reaktionsmischung und dann weiter während der laufenden Veresterungsreaktion kontinuierlich ab. Das erfindungsgemäße Verfahren kann auch nach der in EP-A-0 585 071 beschriebenen Methode durchgeführt werden. Hier wird die Veresterungsreaktion teils bei Atmosphärendruck und teils unter Anwendung eines Vakuums zur schnelleren Austragung des Wassers durchgeführt. Nach Erreichen des angestrebten Umsetzungsgrades wird die Veresterungsreaktion zum Beispiel durch Abkühlen beendet. Das erhaltene Reaktionsprodukt ist bei Raumtemperatur flüssig oder fest. Eine Konfektionierung des bei Raumtemperatur festen Produktes kann man zum Beispiel mit Hilfe einer Schuppenwalze oder eines Kühlbandes durchführen.

Zur Verbindung Ammonium-Hydroxyalkansulfonat, die ebenso wie die anderen Komponenten bekannt und am Markt erhältlich ist, sei noch folgendes gesagt: Ammoniumsalze von Hydroxyalkansulfonsäuren können durch Neutralisation von zum Beispiel Isethionsäure mit Ammoniak oder einem Amin hergestellt werden oder durch Umsetzung eines Amins mit Schwefeldioxid und einem Alkylenoxid in wäßriger Lösung, wie das nachstehende Reaktionsschema mit Trimethylamin als Aminverbindung und Ethylenoxid als Alkylenoxidverbindung zeigt:

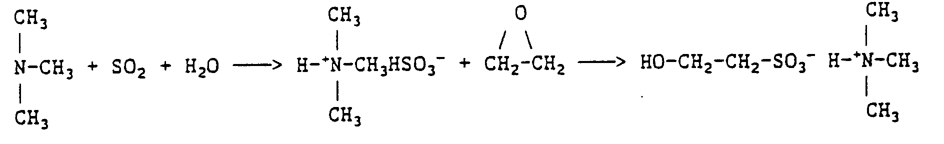

Mit dem erfindungsgemäßen Verfahren, das auch großtechnisch durchführbar ist, können konzentrierte Ammonium-Acyloxyalkansulfonate hergestellt werden. Sie weisen als weitere vorteilhafte Eigenschaften eine im Vergleich zu anderen Salzen bessere Wasserlöslichkeit und ein gut wirkendes Hautgefühl auf. Die erfindungsgemäß erhaltenen Produkte eignen sich deshalb vor allem auch für wäßrige Formulierungen. Durch die Direktveresterung (Direktkondensation) kann auf die Verwendung von Fettsäurechloriden verzichtet werden, die sonst in einem gesonderten Schritt aus Fettsäure hergestellt werden müßten. Die Verwendung von Kosistenzreglern und/oder Verdünnungsmitteln, die in der Regel keine Wertstoffe für zum Beispiel Reinigungsformulierungen und kosmetischen Mitteln darstellen, ist nicht notwendig. Beim erfindungsgemäßen Verfahren besteht also die Reaktionsmischung im wesentlichen lediglich aus Fettsäure, Ammonium-Hydroxyalkansulfonat und Veresterungskatalysator. Das feste oder flüssige Reaktionsprodukt enthält, wie oben erwähnt, im allgemeinen 75 bis 90 Gew.-% Acyloxyalkanammoniumsulfonat, bezogen auf das feste oder flüssige Gesamtprodukt. Die erfindungsgemäß erhaltenen Ammoniumsalze von Acyloxyalkansulfonsäuren entsprechen der nachstehenden Formel 4 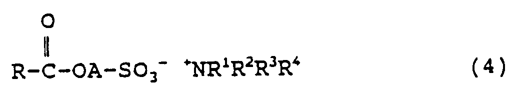 worin R, A und R¹ bis R⁴ die angegebenen Bedeutungen haben.

Die Erfindung wird nun an Beispielen noch näher erläutert. Prozentangaben beziehen sich auf das Gewicht, wenn keine anderen Angaben gemacht sind.

### Beispiel 1

In einem 2-l-Planschliffbecher mit Ankerrührer, absteigender Destillationsbrücke, Innenthermometer und Stickstoffeinleitung werden 401 g (2 mol) Laurinsäure, 511 g von einer wäßrigen 56%igen Ammoniumisethionat-Lösung (das sind 2 mol Ammoniumisethionat) und 1,5 g Zinkoxid vorgelegt. Die Mischung wird auf 190 °C erwärmt und bei dieser Temperatur gehalten. Das in die Mischung eingebrachte und das bei der Direktkondensation gebildete Wasser destilliert kontinuierlich ab. Bei einem Ammoniumlauroylisethionat-Gehalt von 86 % wird die Reaktion abgebrochen und das Reaktionsprodukt zum Erkalten auf ein Blech gegossen. Es besteht im wesentlichen aus 86 % Ammoniumlauroylisethionat, 8 % Laurinsäure und 6 % Ammoniumisethionat.

### Beispiel 2

In einem 3-l-Planschliffbecher mit Ankerrührer, absteigender Destillationsbrücke, Innenthermometer und Stickstoffeinleitung werden 436 g (2 mol) Cocosfettsäure mit einem mittleren Molekulargewicht von 218, 596 g von einer wäßrigen 48%igen Ammoniumisethionat-Lösung (das sind 2 mol Ammoniumisethionat) und 1,5 g Zinkoxid vorgelegt. Die Mischung wird auf 180 °C erwärmt und bei dieser Temperatur gehalten. Das in die Mischung eingebrachte und das bei der Direktkondensation gebildete Wasser destilliert kontinuierlich ab. Bei einem Ammoniumcocoylisethionat-Gehalt von 90 % wird die Reaktion abgebrochen. Die Reaktionsmischung wird auf 145 °C abgekühlt und zum Erkalten auf ein Blech gegossen. Das Endprodukt besteht im wesentlichen aus 90 % Ammoniumcocoylisethionat, 5 % Cocosfettsäure und 5 % Ammoniumisethionat.

### Beispiel 3

In der Einrichtung vom Beispiel 2 werden 533 g (2,6 mol) Cocosfettsäure mit einem mittleren Molekulargewicht von 205, 537 g von einer wäßrigen 53%igen Ammoniumisethionat-Lösung (2 mol Ammoniumisethionat) und 1,6 g Zinkoxid vorgelegt. Die Mischung wird auf 180 °C erwärmt und bei dieser Temperatur gehalten. Das in die Mischung eingebrachte und das bei der Direktveresterung gebildete Wasser destilliert kontinuierlich ab. Bei einem Ammoniumcocoylisethionat-Gehalt von 80 % wird Vakuum (0,5 mbar) angelegt und bei einer Temperatur von weiterhin 180 °C die im Überschuß eingesetzte Fettsäure abdestilliert. Das Reaktionsprodukt wird auf 155 °C abgekühlt und zum Erkalten auf ein Blech gegossen. Das Produkt besteht im wesentlichen aus 88 % Ammoniumcocoylisethionat, 8 % Cocosfettsäure und 4 % Ammoniumisethionat.

### Beispiel 4

In der Einrichtung vom Beispiel 2 werden 521 g (2,6 mol) Laurinsäure, 521 g von einer wäßrigen 55%igen Ammoniumisethionat-Lösung (2 mol Ammoniumisethionat) und 1,6 g Zinkoxid vorgelegt. Die Mischung wird auf 200 °C erwärmt und bei dieser Temperatur gehalten, wobei anwesendes Wasser kontinuierlich abdestilliert. Bei einem Ammoniumlauroylisethionat-Gehait von 75 % wird die Reaktion durch Abkühlen auf Raumtemperatur abgebrochen. Das Reaktionsprodukt besteht im wesentlichen aus 75 % Ammoniumlauroylisethionat, 20 % Laurinsäure und 5 % Ammoniumisethionat.

### Beispiel 5

In der Einrichtung vom Beispiel 2 werden 436 g (2 mol) Cocosfettsäure mit einem mittleren Molekulargewicht von 218, 407 g von einer wäßrigen 53%igen Ammoniumisethionat-Lösung (1,5 mol Ammoniumisethionat) und 1,5 g Zinkoxid vorgelegt. Der Ansatz wird auf 180 °C erwärmt und bei dieser Temperatur gehalten. Das in die Mischung eingebrachte und das bei der Direktkondensation gebildete Wasser destilliert kontinuierlich ab. Bei einem Ammoniumcocoylisethionat-Gehalt von 75 % wird die Reaktion abgebrochen. Das Reaktionsprodukt wird auf 100 °C abgekühlt und zum Erkalten auf ein Blech gegossen. Es besteht im wesentlichen aus 75 % Ammoniumcocoylisethionat, 19 % Cocosfettsäure und 6 % Ammoniumisethionat.

### Beispiel 6

In der Einrichtung vom Beispiel 2 werden 218 g (1 mol) Cocosfettsäure mit einem mittleren Molekulargewicht von 218, 349 g von einer wäßrigen 65%igen Triethylammoniumisethionat-Lösung (1 mol Triethylammoniumisethionat) und 0,8 g Zinkoxid vorgelegt. Die Mischung wird auf 180 °C erwärmt und bei dieser Temperatur gehalten, wobei anwesendes Wasser kontinuierlich abdestilliert. Bei einem Triethylammoniumcocoylisethionat-Gehalt von 87 % wird die Reaktion abgebrochen und das Reaktionsprodukt auf 20 °C abgekühlt. Das flüssige Reaktionsprodukt besteht im wesentlichen aus 87 % Triethylammoniumcocoylisethionat, 6 % Cocosfettsäure und 7 % Triethylammoniumisethionat.

In allen Beispiele wurde der Ammoniumacyloxyisethionat-Gehalt im Reaktionsprodukt mit Hilfe der Epton-Titration und der Fettsäuregehalt durch potentiometrische Titration bestimmt und der Gehalt an Ammoniumisethionat aus dem Umsatz berechnet.

## Patentansprüche

1. Verfahren zur Herstellung von Acyloxyalkansulfonaten mit verbesserten Eigenschaften durch Veresterung von Fettsäuren mit Hydroxyalkansulfonaten, dadurch gekennzeichnet, daß man mindestens eine Fettsäure der Formel 1 RCOOH (1), worin R ein Kohlenwasserstoffrest mit 5 bis 31 Kohlenstoffatomen ist, mit mindestens einem Ammonium-Hydroxyalkansulfonat der Formel 2 HO-A-SO₃⁻ ⁺NR¹R²R³R⁴ (2), worin A ein C₂ bis C₄-Alkylen ist und R¹, R², R³ und R⁴, gleich oder verschieden, Wasserstoff oder eine C₁ bis C₄-Alkylgruppe sind, in Gegenwart eines Veresterungskatalysators und in Abwesenheit eines Konsistenzreglers bei einer Temperatur von höchstens 200 °C unter Entfernen des anwesenden Wassers verestert, wobei ein Produkt mit einem hohen Gehalt an Acyloxyalkanammoniumsulfonat erhalten wird.

2. Verfahren nach Anspruch 1, dadurch gekennzeichnet, daß R ein unverzweigter C₅ bis C₂₁-Alkylrest, ein unverzweigter C₅ bis C₂₁-Alkenylrest oder eine Mischung davon ist, A -CH₂CH₂-, -(CH₂)₃- oder -CH₂CH(CH₃)- ist und R¹ bis R⁴, gleich oder verschieden, H, CH₃ oder C₂H₅ sind.

3. Verfahren nach Anspruch 1, dadurch gekennzeichnet, daß R ein unverzweigter C₅ bis C₂₁-Alkylrest, ein unverzweigter C₅ bis C₂₁-Alkenylrest oder eine Mischung davon ist, A -CH₂CH₂- ist und das Ammoniumion ⁺NR¹R²R³R⁴ ⁺NH₄, ⁺NH(CH₃)₃ oder ⁺NH(C₂H₅)₃ ist.

4. Verfahren nach einem oder mehreren der Ansprüche 1 bis 3, dadurch gekennzeichnet, daß die Veresterung bei einer Temperatur von 170 bis 200 °C durchgeführt wird.

5. Verfahren nach einem oder mehreren der Ansprüche 1 bis 4, dadurch gekennzeichnet, daß die Veresterung bei einer Temperatur von 180 bis 190 °C durchgeführt wird.

6. Verfahren nach einem oder mehreren der Ansprüche 1 bis 5, dadurch gekennzeichnet, daß die Fettsäure und das Hydroxyalkansulfonat im Molverhältnis von 1 : 1 bis 2 : 1 eingesetzt werden.

7. Verfahren nach einem oder mehreren der Ansprüche 1 bis 5, dadurch gekennzeichnet, daß die Fettsäure und das Hydroxyalkansulfonat im Molverhältnis von etwa 1 : 1 eingesetzt werden.

8. Verfahren nach einem oder mehreren der Ansprüche 1 bis 7, dadurch gekennzeichnet, daß als Veresterungskatalysator Zinkoxid in einer Menge von 0,05 bis 2 Gew.-%, bezogen auf Fettsäure und Hydroxyalkansulfonat, eingesetzt wird.

9. Verfahren nach Anspruch 1, dadurch gekennzeichnet, daß man mindestens eine Fettsäure der Formel 1, worin R ein unverzweigter C₅ bis C₂₁-Alkylrest oder ein unverzweigter C₅ bis C₂₁-Alkenylrest oder eine Mischung davon ist, mit mindestens einem Hydroxyalkansulfonat der Formel 2, worin A -CH₂CH₂- und ⁺NR¹R²R³R⁴ ⁺NH₄, ⁺NH(CH₃)₃ oder ⁺NH(C₂H₅)₃ ist, im Molverhältnis von 2 : 1 bis 1 : 1 in Gegenwart von Zinkoxid in einer Menge von 0,05 bis 1 Gew.-%, bezogen auf Fettsäure und Hydroxyalkansulfonat, bei einer Temperatur von 170 bis 200 °C verestert.

10. Verfahren nach Anspruch 9, dadurch gekennzeichnet, daß man die Fettsäure und das Hydroxyalkansulfonat im Molverhältnis von etwa 1 : 1 einsetzte und bei einer Temperatur von 180 bis 190 °C verestert.
